# EUROPÄISCHE PATENTSCHRIFT

(11) **EP 2 083 262 B1**
(45) Veröffentlichungstag und Bekanntmachung des Hinweises auf die Patenterteilung: **07.05.2014**
(21) Anmeldenummer: 08001491.3
(22) Anmeldetag: 28.01.2008
(51) Int. Cl.: G01N 27/12, G01N 33/00

(54) **Resistiver Wasserstoffsensor**
Resistive hydrogen sensor
Capteur d'hydrogène résistif

(43) Veröffentlichungstag der Anmeldung: 29.07.2009
(73) Patentinhaber: Micronas GmbH, 79108 Freiburg i. Br. (DE)
(72) Erfinder: Erdler, Gilbert, Dipl.-Ing., 79100 Freiburg (DE); Reinecke, Holger, Prof. Dr., 79312 Emmendingen (DE); Müller, Claas, Dr., 79104 Freiburg (DE); Frank, Mirko, Dipl.-Ing., 79098 Freiburg (DE)
(74) Vertreter: Koch, Bertram

(56) Entgegenhaltungen:
- JP-A- 2003 279 522
- US-A- 4 953 387
- US-A1- 2004 261 500
- US-A1- 2005 155 858
- WANG ET AL: "Palladium-silver thin film for hydrogen sensing" SENSORS AND ACTUATORS B, ELSEVIER SEQUOIA S.A., LAUSANNE, CH, Bd. 123, Nr. 1, 30. März 2007 (2007-03-30), Seiten 101-106, XP022011183 ISSN: 0925-4005

## Beschreibung

Die Erfindung betrifft einen resistiven Wasserstoffsensor nach dem Oberbegriff von Anspruch 1.

Ein derartiger resistiver Wasserstoffsensor ist aus US-A 4 953 387 bekannt. Er weist auf einem Substrat vier nebeneinander angeordnete Kontaktschichten mit jeweils einem elektrischen Anschluss auf Die Kontaktschichten sind von einer Widerstandsschicht aus Palladium überdeckt, welche sich durchgehend über die Kontaktschichten erstreckt. Die Widerstandsschicht ändert bei Wasserstoffaufnahme ihren elektrischen Widerstand. Die Widerstandsschicht hat Grenzflächen, an denen sie an die Kontaktschichten angrenzt. Um den Einfluss der Kontaktwiderstande auf das Messergebnis zu reduzieren, wird eine sogenannte Vierpunktmessung durchgeführt, bei der in die beiden äußeren Kontaktschichten ein Strom eingespeist wird und an den beiden inneren Kontaktschichten die aus dem Stromfluss resultierende Spannung gemessen wird.

Aus Sakamoto, Y. et al.: "Electrical restistance measurements as a function of composition of palladium-hydrogen(deuterium) systems by a gas phase method", J. Phys.: Condens. Matter 8 (1996), Seiten 3399-3411 ist ferner eine Wasserstoffsensor bekannt, der als Widerstandsschicht eine Palladium-Folie mit einer Dicke von 50-60 µm aufweist, die in Streifen von ca. 2 mm x 5 mm geschnitten ist. Die Widerstandsschicht ist in einem Reaktionsgefäß aus Edelstahl angeordnet, das über Zuleitungen mit einem Wasserstoff- und einem Deuterium-Vorratsbehälter verbunden ist. An die Widerstandsschicht sind Nickeldrähte mit einem Durchmesser von etwa 0,3 mm angeschweißt, welche die Widerstandsschicht mit außerhalb des Reaktionsgefäßes angeordneten elektrischen Anschlüssen verbinden. Als elektrische Isolierung für die Nickeldrähte sind Glasröhrchen vorgesehen, die mit dem Reaktionsgefäß verbunden sind. Bei einer Temperatur von 273 K beträgt der elektrische Widerstand der wasserstoffrelen Palladium-Folie etwa 4,682±0,1 067 mΩ Wird die Palladium-Folie bei Raumtemperatur einer WasserstoffAtmosphäre mit einem Druck von einem bar ausgesetzt, so ändert sich ihr spezifischer Widerstand um circa 80% im Vergleich zu einer wasserstoffreien Palladium-Folie. Der Wasserstoffsensor hat den Nachteil, dass der elektrische Widerstand der Palladium-Folie nur relativ gering ist, so dass der Widerstand der Zuleitungen, welche die Anschlüsse mit der Palladium-Folie verbinden, wesentlich in die Messung mit eingeht. Da der elektrische Widerstand der Zuleitungen mit abnehmendem Leitungsquerschnitt zunimmt, ist eine Miniaturisierung des Wasserstoffsensors nur begrenzt möglich. Wird der Querschnitt der Zuleitungen zu gering dimensioniert, wird der elektrische Widerstand zwischen den Anschlüssen des Wasserstoffsensors im Wesentlichen nur noch durch die Zuleitungen bestimmt. Auch können Temperaturveränderungen, die den elektrischen Widerstand der Palladium-Folie und der Zuleitungen beeinflussen, zu Fehlern bei der Messung führen.

Aus Wang, Min et al.: "Palladium-silver thin film for hydrogen sensing", Sensors and Actuators B 123 (2007), Seiten 101-106 ist außerdem ein Wasserstoffsensor bekannt, der eine Widerstandsschicht aus einer Palladium-Silber-Legierung aufweist, die mittels eines Dünnschichtprozesses auf ein Keramiksubstrat aufgebracht ist. Gegenüber einer Widerstandsschicht aus reinem Palladium ermöglicht die Polladium-Silber-Legierung eine größere Stabilität bei der Wasserstoffeinlagerung. Um den elektrischen Widerstand zu erhöhen, ist die Widerstandsschicht mäanderförmig strukturiert. Ungünstig ist dabei jedoch, dass die Widerstandsschicht einen relativ großen Oberflächenbereich auf dem Substrat bedeckt. Trotz der mäanderförmigen Ausgestaltung ist auch bei diesem Wasserstoffsensor der elektrische Widerstand der Widerstandsschicht noch relativ gering.

Es besteht deshalb die Aufgabe, einen Wasserstoffsensor der eingangs genannten Art zu schaffen, der bei kompakten Abmessungen eine hohe Messempfindlichkeit ermöglicht.

Diese Aufgabe wird mit den Merkmalen des Anpruchs 1 gelöst.

In vorteilhafter Weise ändert sich bei einer Veränderung der Wassersffokonzentratlon zusätzlich zum elektrischen Widerstand der Widerstandsschicht auch der Kontakfwiderstand an der Grenzfläche zwischen der Widerstandsschicht und der Kontaktschicht. Dadurch ergibt sich eine deutliche Veränderung des elektrischen Widerstands zwischen den Anschlüssen des Wasserstoffsensors und somit eine hohe Messempfindlichkeit Der Wasserstoffsensor kann mit aus der Halbleiterfertigungstechnlk bekannten Prozessschritten mit kompakten Abmessungen hergestellt werden, indem die Widerstandsschicht auf ein Substrat aufgebracht wird, beispielsweise ein Siliziumsubstrat. Augrund der im Vergleich zu herkömmlichen resistiven Wasserstoffsensoren erhöhten Empfindlichkeit kann die Widerstands-schicht über Leiterbahnen relativ geringen Querschnitts mit den elektrischen Anschlüssen verbunden sein. Dabei sind die Leiterbahnen bevorzugt auf und/oder in das Substrat integriert. Der Wasserstoffsensor ist dadurch kostengünstig herstellbar und ermöglicht kompakte Abmessungen. Das mindestens eine chemische Element der vierten Nebengruppe kann insbesondere Hafnium, oder Zirkon sein. Der Wasserstoffsensor ermöglicht ein hohes Messsignal, wobei das Gesamtmesssignal der Summe der an den einzelnen Schichtanordnungen auftretenden Teilmesssignale entspricht.

Bei einer bevorzugten Ausführungsform der Erfindung besteht die Kontaktschicht aus Titan oder enthält Titan und/oder eine titanhaltige chemische Verbindung. Mit einer solchen Kontaktschicht kann eine besonders hohe Messempfindlichkeit erzielt werden. Außerdem ist Titan vergleichsweise kostengünstig verfügbar.

Bei einer vorteilhaften Ausgestaltung der Erfindung ist die Widerstandsschicht auf einem Substrat angeordnet, wobei die Kontaktschicht die Widerstandsschicht zumindest bereichsweise derart überdeckt, dass mindestens ein Teilbereich der Widerstandsschicht zwischen der Grenzfläche und dem Substrat angeordnet ist. Die aus der Widerstandsschicht und der Kontaktschicht bestehende Schichtenfolge ermöglicht dann auch bei geringen Schichtdicken eine relativ groBe Grenzfläche zwischen der Widerstandsschicht und der Kontaktschicht.

Zweckmäßigerweise ist die Kontaktschicht von einer Passivierungsschicht überdeckt. Dadurch wird bei einem Kontakt des Wasserstoffsensors mit Luftsauerstoff eine Oxidation der Kontaktschicht vermieden.

Die Widerstandsschicht besteht bevorzugt aus Palladium oder weist eine palladiumhaltige Legierung und/oder eine palladiumhaltige chemische Verbindung auf Der Wasserstoffsensor ermöglicht dann eine noch größereMessempfindlichkeit.

Bei einer anderen vorteilhaften Ausgestaltung der Erfindung enthält die Widerstandsschicht eine zur Einlagerung von Wasserstoff geeignete chemische Verbindung zwischen einem ersten chemischen Element A und einem zweiten chemischen Element B, die vom Typ AB₂ oder AB₅ ist. Auch mit einer solchen Widerstandsschicht kann eine hohe Messempfindlichkeit erreicht werden.

Vorteilhaft ist, wenn die Passivierungsschicht eine metallische Schicht ist, die an einer zweiten Grenzfläche an die Widerstandsschicht angrenzt, und wenn die Passivierungsschicht zwischen den elektrischen Anschlüssen mit der Kontaktschicht und der Widerstandsschicht in Reihe geschaltet ist und vorzugsweise aus Gold besteht. Bei einem Stromfluss zwischen den elektrischen Anschlüssen tritt dann an der zwischen der Widerstandsschicht und der Kontaktschicht gebildeten ersten Grenzfläche und/oder der zwischen der Kontaktschicht und der Passivierungsschicht gebildeten zweiten Grenzfläche ein von der Wasserstoffkonzentration in der Widerstandsschicht abhängiger Spannungsabfall auf Der Wasserstoffsensor ermöglicht dadurch an den elektrischen Anschlüssen ein noch größeres Messsignal.

Bei einer bevorzugten Ausführungsform der Erfindung ist die Kontaktschicht als Dünnfilm ausgebildet, der eine Schichtdicke von weniger als 300 µm, insbesondere weniger als 200 µm und bevorzugt weniger als 100 µm aufweist. Der Wasserstoff sensor kann dann mit Standardprozessen der Halbleiterfertigungstechnik kostengünstig beispielsweise auf einem Substrat aus Silizium hergestellt werden. Der Wasserstoffsensor ermöglicht dadurch sehr kompakte Abmessungen.

Bei einer möglichen Ausführungsform der Erfindung ist zumindest ein Teilbereich der Kontaktschicht zwischen der Widerstandsschicht und dem Substrat angeordnet. Die Widerstandsschicht dient dabei außer zur Generierung des Messsignals auch als Passivierung für die Kontakschicht.

Bei einer bevorzugten Ausgestaltung der Erfindung weist die Widerstandsschicht mindestens einen an die Atmosphäre angrenzenden Oberflächenbereich auf, wobei dieser Oberflächenbereich vorzugsweise seitlich an die zwischen der Widerstandsschicht und der Kontaktschicht befindliche Grenzfläche angrenzt. Der Wasserstoffsensor kann dann zum Detektieren von in der Umgebung befindlichem Wasserstoffgas verwendet werden. Durch eine kontinuierliche Messung des elektrischen Widerstands zwischen den Anschlüssen des Wasserstoffsensors kann eindeutig auf den Wasserstoffpartialdruck in der Umgebung geschlossen werden. Wenn der an die Atmosphäre angrenzende Oberflächenbereich zu der Grenzfläche benachbart ist, kann der Wasserstoff über kurze Diffusionswege von dem Oberflächenbereich zu der Grenzfläche gelangen. Das Messsignal des Wasserstoff sensors reagiert dann schnell auf Änderungen des Wasserstoffpartialdrucks.

Bei einer anderen vorteilhaften Ausgestaltung der Erfindung ist die Widerstandsschicht ein mit einer Brennstoffzelle verbundener Wasserstoffspeicher und die elektrischen Anschlüsse sind mit einer Auswerteeinrichtung verbunden, die als Ladezustandsanzeige für den Wasserstoffspeicher ausgebildet ist. Dabei weist die Auswerteeinrichtung vorzugsweise eine Anzeige für Ladezustand auf Somit kann auf einfache Weise der Ladezustand des Wasserstoffspeichers überprüft und/oder die Menge des in dem Wasserstoffspeicher eingelagerten Wasserstoffs gemessen werden.

Vorteilhaft ist, wenn die Brennstoffzelle einen Schichtstapel, bestehend zumindest aus zwei Elektrodenschichten und einer dazwischen befindlichen Membranschicht aufweist, und wenn die Widerstandsschicht zumindest bereichsweise zwischen diesem Schichtstapel und dem Substrat angeordnet ist. Dadurch ergibt sich eine sehr kompakte Anordnung, bei der die Brennstoffzelle zum Einsparen von Chipfläche auf dem Wasserstoffsensor angeordnet ist. Die Membranschicht ist eine ionenleitende Schicht, bevorzugt eine Polymer-Elektrolytmembran.

Nachfolgend sind Ausführungsbeispiele der Erfindung näher erläutert. Es zeigt:
- Fig. 1: einen Querschnitt durch ein erstes Ausführungsbeispiel des Wasserstoffsensors,
- Fig. 2: eine Aufsicht auf den in Fig. 1 gezeigten Wasserstoffsensor,und
- Fig. 3: eine schematische Darstellung, die Adsorbieren von Wasserstoff an die Oberfläche einer Palladiumschicht und Absorbieren von Wasserstoff die Palladiumschicht des Wasserstoffgassensor zeigt, und
- Fig. 4: einen Querschnitt durch ein zweites, in eine Berennstoffzelle integriertes Ausführungsbeispiel des Wasserstoffsensors, wobei ein an der Brennstoffzelle angeschlossener elektrischer Verbraucher schematisch durch ein Widerstandssymbol dargestellt ist.

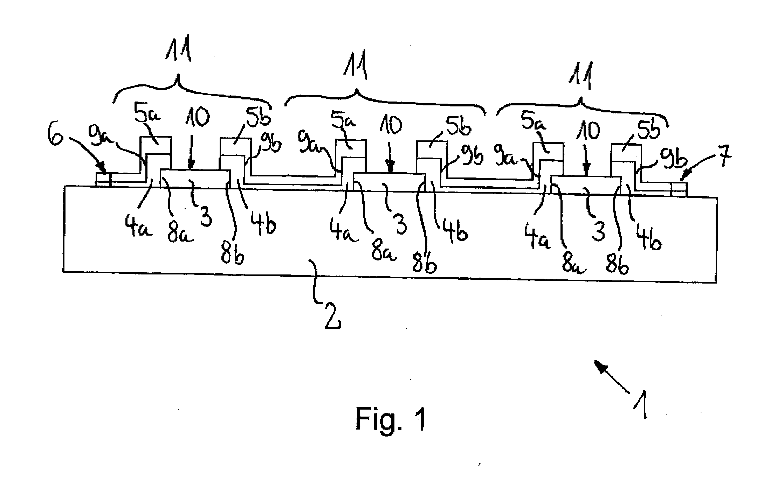

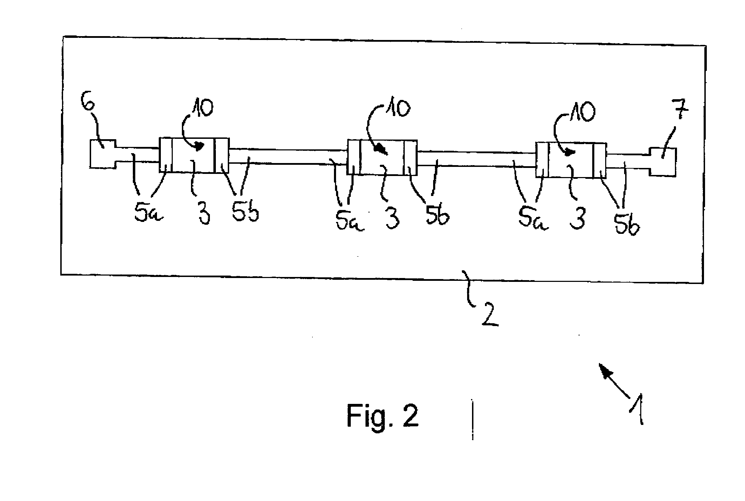

Bei dem in Fig.1 und 2 gezeigten Ausführungsbeispiel sind auf einem Substrat 2 mehrere der Schichtanordnungen 11, jeweils bestehend aus einer Widerstandsschicht 3, einer daran an einer ersten Grenzfläche 8a, 8b angrenzenden Kontaktschicht 4a, 4b und einer an einer zweiten Grenzfläche 9a, 9b an die Kontaktschicht 4a, 4b angrenzenden Passivierungsschicht 5a, 5b vorgesehen. Deutlich ist erkennbar, dass diese Schichtanordnungen 11 zwischen den Anschlüssen 6, 7 derart in Reihe geschaltet sind, dass der von der Stromquelle an die Anschlüsse 6, 7 angelegte elektrische Strom durch alle erste Grenzflächen 8a, 8b und durch mindestens zwei der zweiten Grenzflächen 9a, 9b hindurchfließt.

Die Passivierungsschichten 5a, 5b bestehen aus einem elektrisch leitfähigen Material, bevorzugt einem Edelmetall, vorzugsweise aus Gold und haben jeweils einen Teilbereich, der als elektrischer Anschluss 6, 7 dient. An den Anschlüssen 6, 7 ist eine Stromquelle einer in der Zeichnung nicht näher dargestellten Auswerteeinrichtung angeschlossen. Das Substrat kann ein elektrisch leifähiges Substrat mit Passivierungsschichten, ein Halbleitersubstrat, wie z.B. ein Siliziumsubstrat, oder ein elektrisch isolierendes Substrat, beispielsweise ein Keramiksubstrat oder ein Kunststoffsubstrat sein.

In Fig. 1 und 2 ist erkennbar, dass die Grenzflächen 8a, 8b an voneinander beabstandeten Randbereichen der Widerstandsschicht 3 angeordnet sind, und dass die Kontaktschichten 4a, 4b jeweils einen dieser Randbereiche mit einem Teilbereich überdecken. Ein weiterer Teilbereich jeder Kontaktschicht 4a, 4b ist jeweils direkt auf dem Substrat 2 angeordnet. Zwischen dem die Widerstandsschicht 3 überdeckenden Teilbereich und dem an das Substart 2 angrenzenden Teilbereich weisen die Kontaktschichten 4a, 4b jeweils einen etwa stufen- oder absatzförmigen Verlauf auf.

Die Passivierungsschicht 5a, 5b überdeckt die ihr zugeordnete Kontaktschicht 4a, 4b jeweils vollständig. Dabei folgt die Passivierungsschicht 5a, 5b dem stufen- oder absatzförmigen Verlauf der darunter liegenden Kontaktschicht 4a, 4b. Die Passivierungsschicht 5a, 5b und die Kontaktschicht 4a, 4b sind jeweils als Dünnfilm ausgebildet. Die Schichtdicke der Passivierungsschicht 5a, 5b und der Kontaktschicht 4a, 4b kann beispielsweise etwa 100 µm betragen.

In Fig. 2 ist noch erkennbar, dass die Passivierungsschicht 5a, 5b und die Kontaktschicht 4a, 4b an den Anschlüssen 6, 7 und dem die Widerstandsschicht 3 überdeckenden Teilbereich jeweils eine größer Breite aufweisen als in einem zwischen den Anschlüssen 6, 7 und dem die Widerstandsschicht 3 überdeckenden Teilbereich befindlichen Abschnitt.

Zwischen den Kontaktschichten 4a, 4b hat die Widerstandsschicht 3 einen an die Atmosphäre angrenzenden Oberflächenbereich 10. Dieser Oberflächenbereich 10 grenzt seitilch direkt an die zwischen der Widerstandsschicht 3 und der betreffenden Kontaktschicht 4a, 4b befindliche erste Grenzfläche 8a, 8b an.

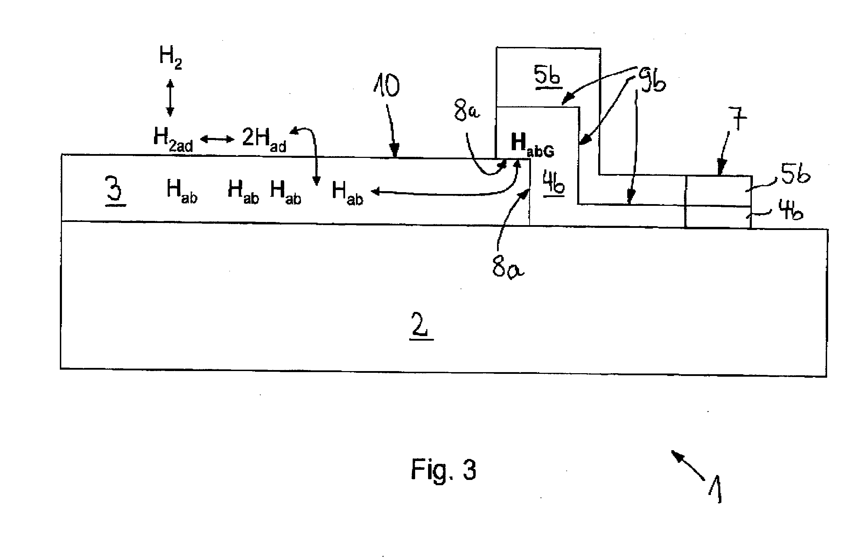

In Fig. 3 ist erkennbar, dass in der Umgebung des Oberflächenbereichs 10 befindliche gasförmige Wasserstoffmoleküle H₂ in Form von Wasserstoffatomen H_{ad} an den Oberflächenbereich 10 adsorbiert werden können. Die Wasserstoffatome H_{ad} werden von der Widerstandsschicht 3 absorbiert und lagern sich in dieser ein. In der Widerstandsschicht 3 diffundieren die absorbierten Wasserstoffatome H_{ab} zu den ersten Grenzflächen 8a, 8b und ggf. den zweiten Grenzflächen 9a, 9b.

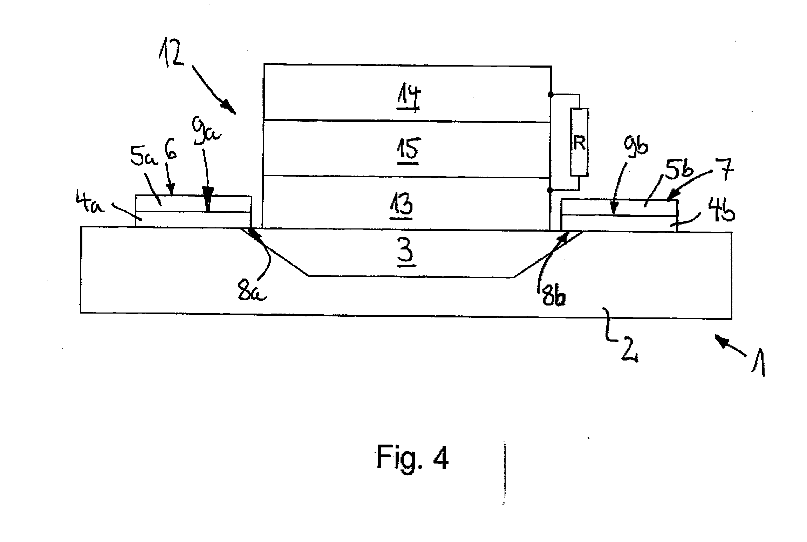

Bei dem in Fig. 4 gezeigten Ausführungsbeispiel ist die Widerstandsschicht als Dickschicht ausgestaltet, die als Wasserstoffspeicher für eine Brennstoffzelle 12 dient Die elektrischen Anschlüsse 6, 7 sind mit einer in der Zeichnung nicht näher dargestellten Auswerteeinrichtung verbunden, die als Ladezustandsanzeige für den Wasserstoffspeicher ausgebildet ist. Die Auswerteeinrichtung kann als elektronische Schaltung in das Substrat 2 integriert sein.

Die Brennstoffzelle ist als Schichtstapel ausgestaltet, der zwei Elektrodenschichten 13, 14 und eine dazwischen befindlichen Membranschicht 15 aufweist. Eine erste Elektrodenschicht 1 3 dient als Kathode und eine zweite, an eine sauerstoffhaltige Atmosphäre angrenzende Elektrodenschicht 14 als Anode. Die Membranschicht 15 ist als Polymer-Elektrolytmembran ausgestaltet. Der Schichtstapel ist auf der Widerstandsschicht 3 zwischen den ersten Grenzflächen 8a, 8b angeordnet. Zwischen den Elektrodenschichten 13, 14 liegt eine elektrische Spannung abgreifbar.

## Patentansprüche

1. Resistiver Wasserstoffsensor (1), der wenigstens zwei elektrische Anschlüsse (6, 7) und mindestens eine Widerstandsschicht (3) aufweist, die bei Wasserstoffaufhahme ihren elektrischen Widerstand ändert, wobei die elektrischen Anschlüsse (6, 7) über die Widerstandsschicht (3) miteinander verbunden sind, wobei die Widerstandsschicht (3) an wenigstens einer Grenzfläche (8a, 8b) an eine Kontaktschicht (4a, 4b) angrenzt, die mindestens ein chemisches Element der vierten Nebengruppe und/oder Kohlenstoff enthält, und wobei die Kontaktschicht (4a, 4b) zwischen den elektrischen Anschlüssen (6, 7) mit der Widerstandsschicht (3) in Reihe geschaltet ist, **dadurch gekennzeichnet, dass** auf dem Substrat (2) mindestens zwei der Schichtanordnungen (11), jeweils bestehend zumindest aus der Widerstandsschicht (3) und der daran angrenzenden Kontaktschicht (4a, 4b) und einer an diese angrenzenden Passivierungsschicht (5a, 5b) angeordnet sind, und dass diese Schichtanordnungen (11) derart zwischen den elektrischen Anschlüssen (6, 7) in Reihe geschaltet sind, dass die Widerstandsschichten (3) voneinander beabstandet sind.

2. Resistiver Wasserstoffsensor (1) nach Anspruch 1, **dadurch gekennzeichnet, dass** die Kontaktschicht (4a, 4b) aus Titan besteht oder Titan und/oder eine titanhaltige chemische Verbindung enthält.

3. Resistiver Wasserstoffsensor (1) nach Anspruch 1 oder 2, **dadurch gekennzeichnet, dass** die Widerstandsschicht (3) auf einem Substrat (2) angeordnet ist, und dass die Kontaktschicht (4a, 4b) die Widerstandsschicht (3) zumindest bereichsweise derart überdeckt, dass mindestens ein Teilbereich der Widerstandsschicht (3) zwischen der Grenzfläche (8a, 8b) und dem Substrat (2) angeordnet ist.

4. Resistiver Wasserstoffsensor (1) nach einem der Ansprüche 1 bis 3, **dadurch gekennzeichnet, dass** die Kontaktschicht (4a, 4b) von einer Passivierungsschicht (5a, 5b) überdeckt ist.

5. Resistiver Wasserstoffsensor (1) nach einem der Ansprüche 1 bis 4, **dadurch gekennzeichnet, dass** die Widerstandsschicht (3) aus Palladium besteht, eine palladiumhaltige Legierung und/oder eine palladiumhaltige chemische Verbindung aufweist.

6. Resistiver Wasserstoffsensor (1) nach einem der Ansprüche 1 bis 5, **dadurch gekennzeichnet, dass** die Widerstandsschicht (3) eine zur Einlagerung von Wasserstoff geeignete chemische Verbindung zwischen einem ersten chemischen Element A und einem zweiten chemischen Element B enthält, die vom Typ AB₂ oder AB₅ ist.

7. Resistiver Wasserstoffsensor (1) nach einem der Ansprüche 1 bis 6, **dadurch gekennzeichnet, dass** die Passivierungsschicht (5a, 5b) eine metallische Schicht ist, die an einer zweiten Grenzfläche (9a, 9b) an die Widerstandsschicht (3) angrenzt, und dass die Passivierungsschicht (5a, 5b) zwischen den elektrischen Anschlüssen (6, 7) mit der Kontaktschicht (4a, 4b) und der Widerstandsschicht (3) in Reihe geschaltet ist und vorzugsweise aus Gold besteht.

8. Resistiver Wasserstoffsensor (1) nach einem der Ansprüche 1 bis 7, **dadurch gekennzeichnet, dass** die Kontaktschicht (4a, 4b) als Dünnfilm ausgebildet ist, der eine Schichtdicke von weniger als 300 µm, insbesondere weniger als 200 µm und bevorzugt weniger als 100 µm aufweist.

9. Resistiver Wasserstoffsensor (1) nach einem der Ansprüche 1 bis 8, **dadurch gekennzeichnet, dass** zumindest ein Teilbereich der Kontaktschicht (4a, 4b) zwischen der Widerstandsschicht (3) und dem Substrat (2) angeordnet ist.

10. Resistiver Wasserstoffsensor (1) nach einem der Ansprüche 1 bis 9, **dadurch gekennzeichnet, dass** die Widerstandsschicht (3) mindestens einen an die Atmosphäre angrenzenden Oberflächenbereich (10) aufweist, und dass dieser Oberflächenbereich (10) vorzugsweise seitlich an die zwischen der Widerstandsschicht (3) und der Kontaktschicht (4a, 4b) befindliche erste Grenzfläche (8a, 8b) angrenzt.

11. Resistiver Wasserstoffsensor (1) nach einem der Ansprüche 1 bis 10, **dadurch gekennzeichnet, dass** die Widerstandsschicht (3) ein mit einer Brennstoffzelle (12) verbundener Wasserstoffspeicher ist und dass die elektrischen Anschlüsse (6, 7) mit einer Auswerteeinrichtung verbunden sind, die als Ladezustandsanzeige für den Wasserstoffspeicher ausgebildet ist.

12. Resistiver Wasserstoffsensor (1) nach einem der Ansprüche 1 bis 11, **dadurch gekennzeichnet, dass** die Brennstoffzelle (12) einen Schichtstapel, bestehend zumindest aus zwei Elektrodenschichten (13, 14) und einer dazwischen befindlichen Membranschicht (15) aufweist, und dass die Widerstandsschicht (3) zumindest bereichsweise zwischen diesem Schichtstapel und dem Substrat (2) angeordnet ist.

## Claims

1. Resistive hydrogen sensor (1), which comprises at least two electrical terminals (6, 7) and at least one resistance layer (3), which changes its electrical resistance when absorbing hydrogen, wherein the electrical terminals (6, 7) are connected together by way of the resistance layer (3), wherein the resistance layer (3) adjoins at least one boundary surface (8a, 8b) at a contact layer (4a, 4b), which contains at least one chemical element of the fourth coset and/or carbon, and wherein the contact layer (4a, 4b) is connected in series between the electrical terminals (6, 7) with the resistance layer (3), **characterised in that** at least two of the layer arrangements (11), which each consist at least of the resistance layer (3) and the contact layer (4a, 4b) adjoining thereat and a passivating layer (5a, 5b) adjoining thereat, are arranged on the substrate (2), and that these layer arrangements (11) are so connected in series between the electrical terminals (6, 7) that the resistance layers (3) are spaced from one another.

2. Resistive hydrogen sensor (1) according to claim 1, **characterised in that** the contact layer (4a, 4b) consists of titanium or contains titanium and/or a chemical compound with titanium content.

3. Resistive hydrogen sensor (1) according to claim 1 or 2, **characterised in that** the resistance layer (3) is arranged on a substrate (2) and that the contact layer (4a, 4b) so covers the resistance layer (3) at least in a region that at least one sub-region of the resistance layer (3) is arranged between the boundary surface (8a, 8b) and the substrate (2).

4. Resistive hydrogen sensor (1) according any one of claims 1 to 3, **characterised in that** the contact layer (4a, 4b) is covered by a passivating layer (5a, 5b).

5. Resistive hydrogen sensor (1) according to any one of claims 1 to 4, **characterised in that** the reistance layer (3) consists of palladium, an alloy with palladium content and/or a chemical compound with palladium content.

6. Resistive hydrogen sensor (1) according to any one of claims 1 to 5, **characterised in that** the resistance layer (3) contains a chemical compound, which is suitable for storage of hydrogen, between a first chemical element A and a second chemical element B, which is of the type AB₂ or AB₃.

7. Resistive hydrogen sensor (1) according to any one of claims 1 to 6, **characterised in that** the passivating layer (5a, 5b) is a metallic layer which adjoins a second boundary surface (9a, 9b) at the resistance layer (3) and that the passivating layer (5a, 5b) is connected between the electrical terminals (6, 7) in series with the contact layer (4a, 4b) and the resistance layer (3) and preferably consists of gold.

8. Resistive hydrogen sensor (1) according to any one of claims 1 to 7, **characterised in that** the contact layer (4a, 4b) is formed as a thin film having a layer thickness of less than 300 microns, particularly less than 200 microns and preferably less than 100 microns.

9. Resistive hydrogen sensor (1) according to any one of claims 1 to 8, **characterised in that** at least a sub-region of the contact layer (4a, 4b) is arranged between the resistance layer (3) and the substrate (2).

10. Resistive hydrogen sensor (1) according to any one of claims 1 to 9, **characterised in that** the resistance layer (3) has at least one surface region (10) adjoining the atmosphere and that this surface region (10) preferably laterally adjoins the first boundary surface (8a, 8b) disposed between the resistance layer (3) and the contact layer (4a, 4b).

11. Resistive hydrogen sensor (1) according to any one of claims 1 to 10, **characterised in that** the resistance layer (3) is a hydrogen store connected with a fuel cell (12) and that the electrical terminals (6, 7) are connected with an evaluating device constructed as a charge state indicator for the hydrogen store.

12. Resistive hydrogen sensor (1) according to any one of claims 1 to 11, **characterised in that** the fuel cell (12) comprises a layer stack consisting of at least two electrode layers (13, 14) and a membrane layer (15) disposed therebetween and that the resistance layer (3) is arranged at least in a region between this layer stack and the substrate (2).

## Revendications

1. Capteur d'hydrogène résistif (1), qui présente au moins deux raccords électriques (6, 7) et au moins une couche de résistance (3), qui modifie sa résistance électrique en cas de capture d'hydrogène, dans lequel les raccords électriques (6, 7) sont reliés l'un à l'autre par la couche de résistance (3), dans lequel la couche de résistance (3) est adjacente par au moins une face limite (8a, 8b) à une couche de contact (4a, 4b), qui contient au moins un élément chimique du quatrième sous-groupe et/ou du carbone, et dans lequel la couche de contact (4a, 4b) est montée en série avec la couche de résistance (3) entre les contacts électriques (6, 7), **caractérisé en ce qu'**au moins deux des agencements de couches (11), dont chacun est composé au moins de la couche de résistance (3) et de la couche de contact (4a, 4b) adjacente à celle-ci et d'une couche de passivation (5a, 5b) adjacente à celle-ci, sont disposés sur le substrat (2) et **en ce que** ces agencements de couches (11) sont montés en série entre les raccords électriques (6, 7), de telle manière que les couches de résistance (3) soient espacées l'une de l'autre.

2. Capteur d'hydrogène résistif (1) selon la revendication 1, **caractérisé en ce que** la couche de contact (4a, 4b) se compose de titane ou contient du titane et/ou un composé chimique contenant du titane.

3. Capteur d'hydrogène résistif (1) selon la revendication 1 ou 2, **caractérisé en ce que** la couche de résistance (3) est disposée sur un substrat (2) et **en ce que** la couche de contact (4a, 4b) recouvre au moins localement la couche de résistance (3), de telle manière qu'au moins une région partielle de la couche de résistance (3) soit disposée entre la face limite (8a, 8b) et le substrat (2).

4. Capteur d'hydrogène résistif (1) selon l'une quelconque des revendications 1 à 3, **caractérisé en ce que** la couche de contact (4a, 4b) est recouverte d'une couche de passivation (5a, 5b).

5. Capteur d'hydrogène résistif (1) selon l'une quelconque des revendications 1 à 4, **caractérisé en ce que** la couche de résistance (3) se compose de palladium, présente un alliage contenant du palladium et/ou un composé chimique contenant du palladium.

6. Capteur d'hydrogène résistif (1) selon l'une quelconque des revendications 1 à 5, **caractérisé en ce que** la couche de résistance (3) contient un composé chimique convenant pour accumuler de l'hydrogène entre un premier élément chimique A et un deuxième élément chimique B, ce composé étant du type AB2 ou AB5.

7. Capteur d'hydrogène résistif (1) selon l'une quelconque des revendications 1 à 6, **caractérisé en ce que** la couche de passivation (5a, 5b) est une couche métallique, qui est adjacente par une deuxième face limite (9a, 9b) à la couche de résistance (3), et **en ce que** la couche de passivation (5a, 5b) est montée en série avec la couche de contact (4a, 4b) et la couche de résistance (3) entre les raccords électriques (6, 7) et est, de préférence, composée d'or.

8. Capteur d'hydrogène résistif (1) selon l'une quelconque des revendications 1 à 7, **caractérisé en ce que** la couche de contact (4a, 4b) est réalisée sous la forme d'un film mince, qui présente une épaisseur de couche de moins de 300 µm, en particulier de moins de 200 µm et, de préférence, de moins de 100 µm.

9. Capteur d'hydrogène résistif (1) selon l'une quelconque des revendications 1 à 8, **caractérisé en ce qu'**au moins une région partielle de la couche de contact (4a, 4b) est disposée entre la couche de résistance (3) et le substrat (2).

10. Capteur d'hydrogène résistif (1) selon l'une quelconque des revendications 1 à 9, **caractérisé en ce que** la couche de résistance (3) présente au moins une zone de surface (10) adjacente à l'atmosphère et **en ce que** cette zone de surface (10) est de préférence adjacente latéralement à la première face limite (8a, 8b) se trouvant entre la couche de résistance (3) et la couche de contact (4a, 4b).

11. Capteur d'hydrogène résistif (1) selon l'une quelconque des revendications 1 à 10, **caractérisé en ce que** la couche de résistance (3) est un accumulateur d'hydrogène relié à une pile à combustible (12) et **en ce que** les raccords électriques (6, 7) sont reliés à un dispositif d'exploitation, qui est réalisé sous la forme d'un affichage de l'état de charge pour l'accumulateur d'hydrogène.

12. Capteur d'hydrogène résistif (1) selon l'une quelconque des revendications 1 à 11, **caractérisé en ce que** la pile à combustible (12) présente un empilement de couches, composé au moins de deux couches d'électrode (13, 14) et d'une couche de membrane (15) se trouvant entre celles-ci et **en ce que** la couche de résistance (3) est au moins localement disposée entre cet empilement de couches et le substrat (2).
